# EUROPEAN PATENT APPLICATION

(11) **EP 1 690 562 A1**
(43) Date of publication of application: **16.08.2006**
(21) Application number: 04818958.3
(22) Date of filing: 18.11.2004
(51) Int. Cl.: A61M 5/34

(54) **SYRINGE NEEDLE**

(30) Priority: 21.11.2003 JP 2003428312
(71) Applicant: TSK Laboratory Japan, Tochigi-shi, Tochigi 328-0012 (JP)
(72) Inventor: KAWASHIMA, Yukio c/o TSK Laboratory Japan, Tochigi-shi, Tochigi 328-0012 (JP)
(74) Representative: Reininger, Jan Christian
(86) International application number: PCT/JP2004/017188
(87) International publication number: WO 2005/049119

(57) **Abstract**

The present invention provides an injection needle capable of preventing needle bending and needle breakage, and being easily manufactured at low cost.

According to the present invention, the needle cover 4 provided movably in the axial direction of the needle body 3 is included, and when the cap 5 covering the needle body 3 is detached from the hub 2, the locking portions 5c provided at the cap 5 are locked at the first locking portion 4a of the needle cover 4 and the needle cover 4 moves to the tip end side of the needle body 3 with the cap 5, so that the tip end side of the needle body 3 is covered with the needle cover 4. Therefore, the external force in the bending direction is not directly applied to the needle body 3 when the cap 5 is detached or after it is detached. Since the needle cover 4 can be moved to the tip end side of the needle body 3 by locking of the cap 5 and the needle cover 4, it is not necessary to use a spring for moving the needle cover 4 to the tip end side of the needle body 3, or to form the needle cover 4 into an extendable and retractable complicated shape.

## Description

### TECHNICAL FIELD

The present invention relates to an injection needle used in injection of drug solutions, blood collection, dialysis and the like.

### BACKGROUND ART

Generally, when an injection needle is inserted into a patient, the skin of the patient is torn by the needle point and pushed to open by the thickness of the needle tube, and therefore, the patient is give pain in many cases. Thus, by making the outside diameter of the needle tube small, the pain of the patient can be relieved, but the needle tube easily bends as the needle tube becomes thinner, and therefore, when those unskilled in medical activity uses injectors, for example, when patients inject drug solutions such as insulin by themselves, useless external force in a bending direction is applied to needles on detaching the caps of the injection needles, thus causing the fear of occurrence of needle bending and needle breakage.

Thus, as an injection needle to prevent needle bending and needle breakage, the one which has a cylindrical needle cover movable in an axial direction of the needle body attached thereon, with the needle cover biased in a needle point direction by a spring is known (for example, see Patent Document 1). As another injection needle, the one that includes an accordion-shaped needle cover extendable and retractable in an axial direction of the needle body, so as to cover the needle body by extending the needle cover is known (for example, see Patent Document 2).

However, the former has the problem that the number of components becomes large by the spring in use, which makes the manufacturing cost high. The latter has the problem that to extend and retract the needle cover itself, the needle cover has the complicated accordion shape, and work at the time of manufacture is not easy.
Patent Document 1: Japanese Patent Publication 3-139363
Patent Document 2: Japanese Patent Publication 6-16295

### DISCLOSURE OF THE INVENTION

The present invention is made in view of the above described problems, and it is an object of the present invention to provide an injection needle capable of preventing needle bending and needle breakage and being manufactured easily at low cost.

In order to achieve the above described object, in an injection needle, which includes a fitting member fitted to a parenteral solution discharge port of an injector body, a needle body held by the fitting member, and a cap detachably attached to the fitting member to cover the needle body, and is used by fitting the fitting member with the cap attached thereon to the injector body, and detaching the cap from the fitting member, the present invention includes a cover member that is provided movably in an axial direction of the aforesaid needle body, exposes a tip end side of the needle body by moving to a base end side of the needle body, and covers the tip end side of the needle body by moving to the tip end side of the needle body, and the aforesaid cap is provided with a locking portion that is locked at the cover member to move the cover member to the tip end side of the needle body with the cap.

Thereby, when the cap is detached from the fitting member, the locking portion of the cap is locked at the cover member, and the cover member moves to the tip end side of the needle body with the cap, so that the tip end side of the needle body is covered with the cover member. Therefore, an external force in the bending direction is not directly applied to the needle body when the cap is detached or after it is detached. In addition, it is not necessary to use a spring for moving the cover member to the tip end side of the needle body, or to form the cover member into an extendable and retractable complicated shape.

According to the injection needle of the present invention, when the cap is detached, the tip end side of the needle body is covered with the cover member. Therefore, an external force in the bending direction is not directly applied to the needle body when the cap is detached or after it is detached, and needle bending and needle breakage can be then reliably prevented. In this case, the cover member can be moved to the tip end side of the needle body by locking of the cap and the cover member, and therefore, it is not necessary to use a spring for moving the cover member to the tip end side of the needle body, or to form the cover member into an extendable and retractable complicated shape, thus making it possible to manufacture the injection needle easily at low cost.

### BRIEFLY DESCRIBE OF THE DRAWINGS

Figure 1 is a perspective view of an injector to which an injection needle of the present invention is fitted;
Figure 2 is a sectional side view of a needle cover and a hub;
Figure 3 is a sectional side view of a cap;
Figures 4 is perspective views showing fitting steps of the injection needle;
Figures 5 is sectional side views showing fitting steps of the injection needle;
Figures 6 is sectional side views showing fitting steps of the injection needle;
Figure 7 is a sectional side view showing a centesis operation into a human body; and
Figures 8 is perspective views showing fitting steps of the cap.

### DESCRIPTION OF SYMBOLS

1 ... injector body, 1a ... parenteral solution discharge port, 2 ... hub, 2a ... extended portion, 3 ... needle body, 4 ... needle cover, 4a ... first locking portion, 5 ... cap, 5c ... locking portion.

### BEST MODE FOR CARRYING OUT THE INVENTION

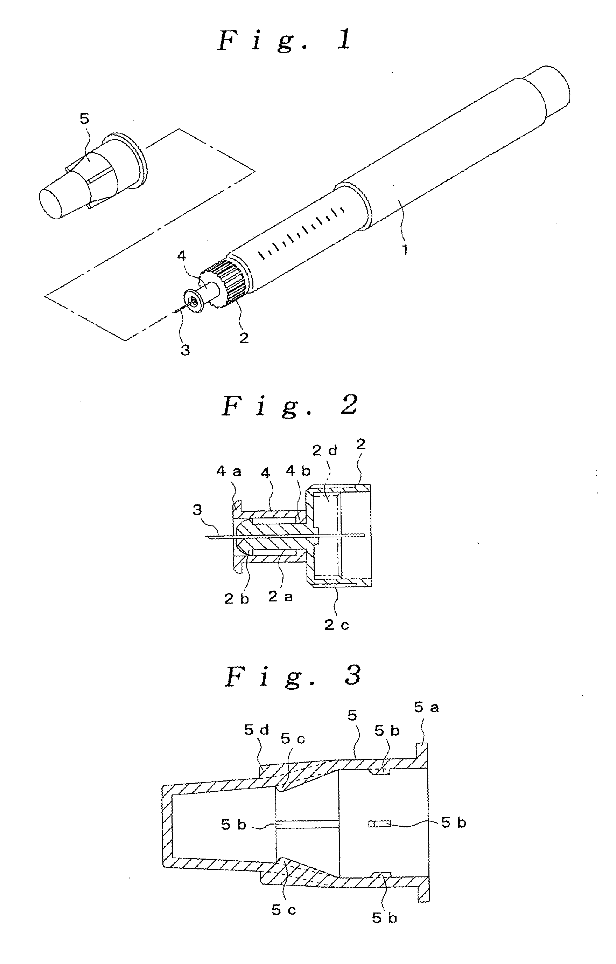

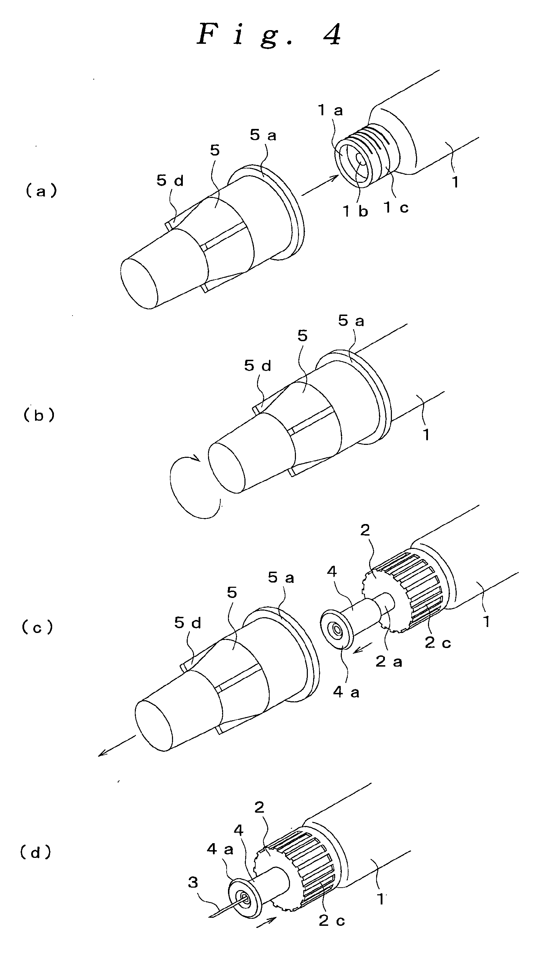

An injection needle shown in Figures 1 to 8 includes a hub 2 as a fitting member fitted to an injector body 1, a needle body 3 held by the hub 2, a needle cover 4 as a cover member provided movably in an axial direction of the needle body 3, and a cap 5 detachably attached to the hub 2 to cover the needle body 3.

The injector body 1 has a parenteral solution discharge port 1a at one end, and a parenteral solution A such as insulin is charged in an inside thereof. The parenteral solution discharge port 1a is closed by a rubber member 1b through which the needle body 3 is capable of penetrating, and a screw portion 1c for fitting the hub 2 is formed on an outer peripheral surface of the parenteral solution discharge port 1a.

The hub 2 is constituted of a cylindrical molded product of a synthetic resin with one end surface closed, and is provided with an extended portion 2a extending in the axial direction at the center of its one end surface. The extended portion 2a extends in the needle point direction to cover the needle body 3 from the one end surface of the hub 2, and the needle body 3 is held by the extended portion 2a. A locking portion 2b which is locked at the needle cover 4 is provided at a tip end side of the extended portion 2a, and the locking portion 2b is formed to expand outward in the diameter direction. A plurality of ribs 2c extending in the axial direction is projectingly provided to be spaced from each other in a circumferential direction on the outer peripheral surface of the hub 2, and a screw portion 2d (simplified in the drawings) capable of being screwed onto the screw portion 1c of the injector body 1 is formed on an inner peripheral surface of the hub 2.

The needle body 3 is constituted of a metal tube with a tip end of the metal tube formed to be sharp, and has its tip end side and base end side projected outward from the extended portion 2a of the hub 2, respectively.

The needle cover 4 is constituted of a cylindrical molded product of a synthetic resin with both ends of the product opened, and is engaged with the extended portion 2a of the hub 2 movably in the axial direction. A first locking portion 4a which is locked at the cap 5 is provided at one end side (tip end side of the needle body 3) in the axial direction of the needle cover 4, and the first locking portion 4a is formed into a flange shape to project outward in the diameter direction. The needle cover 4 has its inside diameter formed to be larger than the outside diameter of the extended portion 2a, and is provided with a second locking portion 4b which is locked at the locking portion 2b of the extended portion 2a at the other end side in its axial direction (base end side of the needle body 3), and the second locking portion 4b is formed to project inward in the diameter direction. Namely, the needle cover 4 moves to the base end side of the needle body 3 along the extended portion 2a to expose the tip end side of the needle body 3, and moves to the tip end side of the needle body 3 to cover the tip end side of the needle body 3. When the needle cover 4 moves to the tip end side of the needle body 3, the second locking portion 4b is locked at the locking portion 2b of the extended portion 2a in the axial direction, so that movement of the needle cover 4 in the needle point direction is restricted.

The cap 5 is constituted of a molded product of a synthetic resin with one end opened, and is provided with a flange portion 5a at its one end opening edge. A plurality of ribs 5b extending in the axial direction is projectingly provided to be spaced in the circumferential direction from each another at one end side of an inner peripheral surface of the cap 5, and each of the ribs 5b is formed to be capable of being locked at the rib 2c of the hub 2 in the circumferential direction. Locking portions 5c capable of being locked at the first locking portion 4a of the needle cover 4 are projectingly provided equidistantly at four spots in total in the circumferential direction at the other end side of the inner peripheral surface of the cap 5. In this case, the space between the locking portions 5c opposed to each other in the diameter direction of the cap 5 is formed to be a little smaller than the outside diameter of the first locking portion 4a of the needle cover 4, and by forcefully moving the cap 5 in the axial direction, each of the locking portions 5c is elastically deformed in the diameter direction to ride over the first locking portion 4a of the needle cover 4. A plurality of ribs 5d extending in the axial direction is projectingly provided to be spaced from each other in the circumferential direction on the outer peripheral surface of the cap 5.

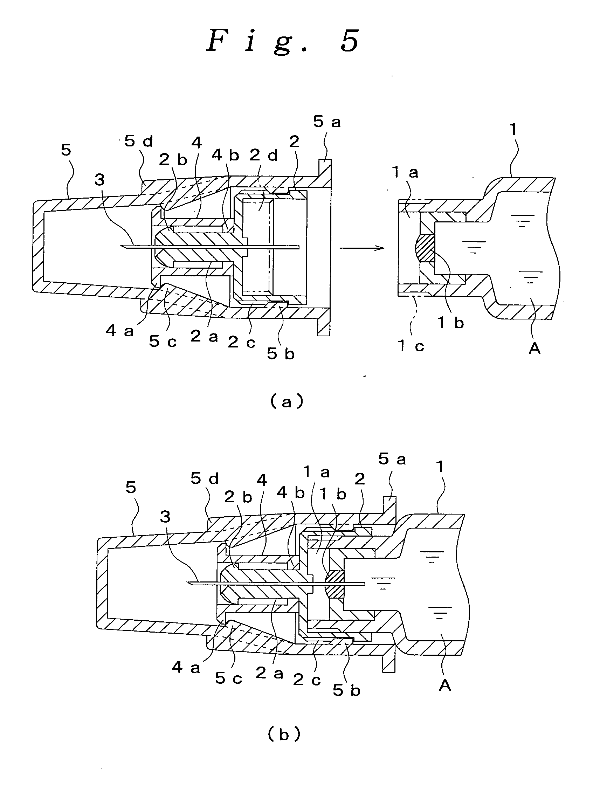

The injection needle constituted of the above construction is accommodated in the cap 5 with the hub 2, the needle body 3 and the needle cover 4 assembled to each other as shown in Figure 5(a), and the cap 5 is sealed with a lid not shown that covers the opening. In this case, each of the locking portions 5c of the cap 5 is located at the base end side of the needle body 3 with respect to the first locking portion 4a of the needle cover 4.

Here, when the above described injection needle is used, the opening of the cap 5 is opened by removing the lid not shown, and the hub 2 is fitted to the parenteral solution discharge port 1a of the injector body 1 with the cap 5 attached as shown in Figure 5(b). In this case, the screw portion 2d of the hub 2 is screwed onto the screw portion 1c of the injector body 1 by rotating the cap 5 around the axis, and thereby, the hub 2 is fitted to the injector body 1. On this occasion, the rib 5b of the cap 5 is locked at the rib 2c of the hub 2 in the circumferential direction, whereby the cap 5 and the hub 2 are integrally rotated, and each of the ribs 5d on the outer peripheral surface of the cap 5 serves as non-slip for the finger tips gripping the cap 5.

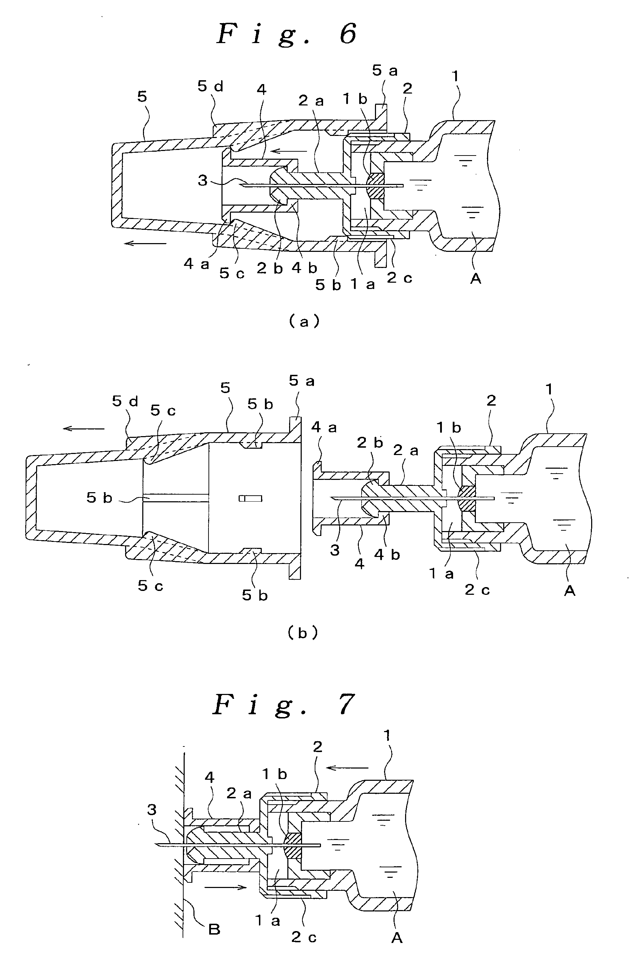

Next, when the cap 5 is moved in the needle point direction as shown in Figure 6(a), the needle cover 4 moves in the needle point direction with the cap 5 because each of the locking portions 5c of the cap 5 is locked at the first locking portion 4a of the needle cover 4 from the base end side of the needle body 3, and the tip end side of the needle body 3 is then covered with the needle cover 4. When the force in the needle point direction is further applied to the cap 5, each of the locking portions 5c of the cap 5 rides over the first locking portion 4a of the needle cover 4, and the cap 5 is detached, as shown in Figure 6(b). Since on this occasion, the tip end side of the needle body 3 is covered with the needle cover 4, the cap 5 does not directly contact the needle body 3 even if the cap 5 is to be contacted to it erroneously when detached, and an external force in the bending direction is not applied to the needle body 3. The needle body 3 is covered with the needle cover 4, and therefore, the finger tips do not directly contact the needle body 3.

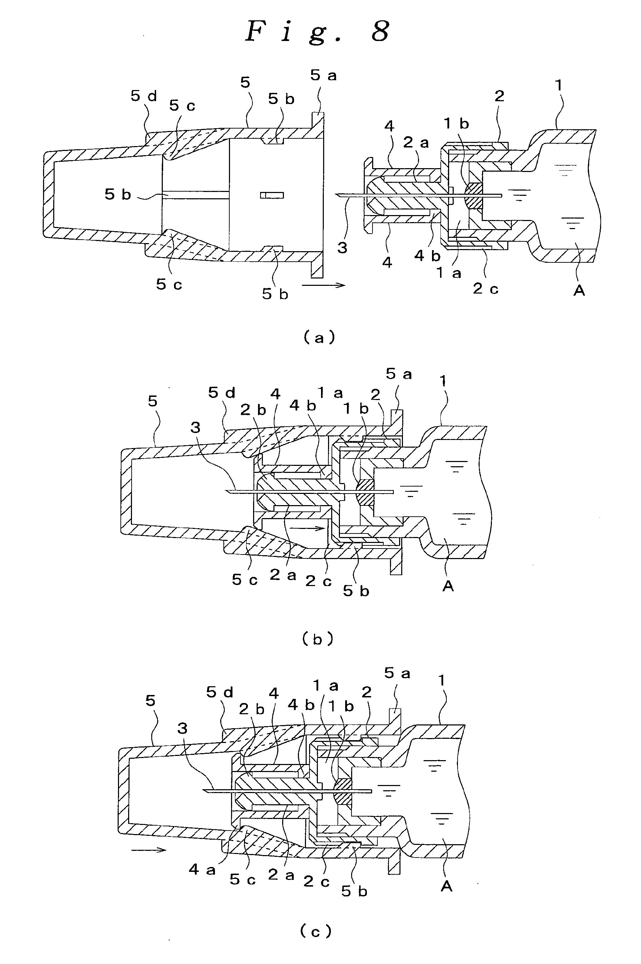

When the tip end of the needle body 3 is inserted into a human body B of a patient as shown in Figure 7 after the cap 5 is detached, the needle cover 4 relatively moves with respect to the extended portion 2a while inhibited from moving to the human body B side by abutment on the human body B, and the needle body 3 is inserted into the human body B. Thereby, the drug solution A in the injector body 1 is dischargeable into the human body B from the needle body 3. After completion of the injection, the needle cover 4 is moved in the needle point direction to cover the tip end side of the needle body 3, and thereby, the safety is ensured.

When the cap 5 is to be attached after use, by putting the cap 5 on the needle body 3 side as shown in Figure 8(a), each of the locking portions 5c of the cap 5 is locked at the first locking portion 4a of the needle cover 4 from the tip end side of the needle body 3, and the needle cover 4 moves in the base end direction of the needle body 3 with the cap 5 to abut on one end surface of the hub 2 as shown in Figure 8(b). When the force in the base end direction of the needle body 3 is further applied to the cap 5, each of the locking portions 5c of the cap 5 is elastically deformed outward in the diameter direction of the cap 5 to ride over the first locking portion 4a of the needle cover 4 as shown in Figure 8(c), and thereby the cap 5 is attached.

When the used injection needle is to be discarded, the cap 5 is rotated in the opposite direction from that at the time of fitting the cap 5 to release the screwed state of the hub 2 and the injector body 1, and thereby, the cap 5 is detached with the hub 2, the needle body 3 and the needle cover 4 accommodated in the cap 5. On this occasion, each of the locking portions 5c of the cap 5 is located at the base end side of the needle body 3 with respect to the first locking portion 4a of the needle cover 4, therefore, the hub 2, the needle body 3 and the needle cover 4 do not fall off the cap 5 due to locking of the first locking portion 4a and each of the locking portions 5c.

As described above, according to the injection needle of the embodiment, the needle cover 4 provided movably in the axial direction of the needle body 3 is included, and when the cap 5 covering the needle body 3 is detached from the hub 2, the locking portions 5c provided at the cap 5 are locked at the first locking portion 4a of the needle cover 4, and the needle cover 4 moves to the tip end side of the needle body 3 with the cap 5, so that the tip end side of the needle body 3 is covered with the needle cover 4. Therefore, an external force in the bending direction is not directly applied to the needle body 3 when the cap 5 is detached or after it is detached, and needle bending and needle breakage can be reliably prevented. Also the needle cover 4 can be moved to the tip end side of the needle body 3 by locking of the cap 5 and the needle cover 4, it is not necessary to use a spring for moving the needle cover 4 to the tip end side of the needle body 3, or to form the needle cover 4 into an extendable and retractable complicated shape, and the injection needle can be easily manufactured at low cost.

Further, since the needle cover 4 is formed into a cylindrical shape and engaged with the extended portion 2a provided at the hub 2 movably in the axial direction, the needle cover 4 can be reliably moved in the axial direction of the needle body 3 due to the simple structure, and work at the time of manufacture can be performed extremely easily.

Also the locking portion 5c of the cap 5 is formed to ride over the first locking portion 4a of the needle cover 4 in the axial direction due to the elastic deformation, the locking operation and the locking releasing operation of the cap 5 and the needle cover 4 can be made easily, and operability can be enhanced.

In this case, the first locking portion 4a of the needle cover 4 is formed into a flange shape to project in the diameter direction of the needle cover 4, and the locking portions 5c of the cap 5 are projectingly provided to be spaced from each other in the circumferential direction at a plurality of spots of the inner peripheral surface of the cap 5. Therefore, the locking portions 5c of the cap 5 can be locked in any locations in the circumferential direction of the first locking portion 4a of the needle cover 4, and the cap 5 and the needle cover 4 can be reliably locked.

## Claims

1. An injection needle which comprises a fitting member fitted to a parenteral solution discharge port of an injector body, a needle body held by the fitting member, and a cap detachably attached to the fitting member to cover the needle body, and is used by fitting the fitting member with the cap attached thereon to the injector body and detaching the cap from the fitting member, comprising:
a cover member that is provided movably in an axial direction of said needle body, exposes a tip end side of the needle body by moving to a base end side of the needle body, and covers the tip end side of the needle body by moving to the tip end side of the needle body,
wherein said cap is provided with a locking portion that is locked at the cover member to move the cover member to the tip end side of the needle body with the cap.

2. The injection needle according to claim 1, wherein
said fitting member is provided with an extended portion extending in the axial direction toward the tip end side of the needle body; and
said cover member is formed into a cylindrical shape and engaged with the extended portion movably in the axial direction.

3. The injection needle according to claim 1 or 2, wherein
a locking portion that locks the locking portion of the cap is provided at a predetermined position in the axial direction of said cover member; and
the locking portion of the cap is formed to ride over the locking portion of the cover member in the axial direction by elastic deformation.

4. The injection needle according to claim 3, wherein
the locking portion of said cover member is formed into a flange shape to project in a diameter direction of the cover member; and
the locking portions of said cap are projectingly provided to be spaced from each other in a circumferential direction at a plurality of spots on an inner peripheral surface of the cap.
